# EUROPEAN PATENT APPLICATION

(11) **EP 4 706 642 A1**
(43) Date of publication of application: **11.03.2026**
(21) Application number: 25200829.7
(22) Date of filing: 08.09.2025
(51) Int. Cl.: A61K 9/48, A61K 31/495

(54) **A SOLID ORAL COMPOSITION COMPRISING CARIPRAZINE HYDROCHLORIDE**

(30) Priority: 10.09.2024 TR 2024011970
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); IPEK, Celaleddin, Istanbul (TR); PEHLIVAN AKALIN, Nur, Istanbul (TR); GENCAL BOZKURT, Aynur, Istanbul (TR); ARMUT, Merve, Istanbul (TR); OZTAS, Bora, Istanbul (TR); KUCUKGUL, Ayse, Istanbul (TR)
(74) Representative: Genç Ilhan, Oznur

(57) **Abstract**

The present invention relates to a solid oral composition comprising cariprazine hydrochloride and at least one pharmaceutically acceptable excipient, wherein cariprazine hydrochloride has a d (0.9) particle size between 30 µm and 1 µm. The present invention also relates to a simple, rapid, cost effective, time-saving and industrially convenient method of preparing the formulation.

## Description

### Field of the Invention

The present invention relates to a solid oral composition comprising cariprazine hydrochloride and at least one pharmaceutically acceptable excipient, wherein cariprazine hydrochloride has a d (0.9) particle size between 30 µm and 1 µm. The present invention also relates to a simple, rapid, cost effective, time-saving and industrially convenient method of preparing the formulation.

### Background of the Invention

Schizophrenia is a lifelong disabling psychiatric disorder with a reported worldwide prevalence of about 1%, including 3.2 million Americans. The disorder usually manifests during adolescence or in young adulthood; the cardinal symptoms fall into three domains - positive symptoms, such as delusions and hallucinations, negative symptoms, such as lack of drive and social withdrawal, and cognitive symptoms, such as problems with attention and memory. These lead to social and occupational dysfunction, which inevitably have a profound effect on the family and the place of the affected individual in wider society. In addition to psychiatric symptoms, patients with schizophrenia are at greater risk for medical comorbidities than the general population.

Drug treatment with dopamine antagonists is the cornerstone of schizophrenia management both during the acute as well as the residual phase. Current guidelines recommend atypical antipsychotics, including risperidone, olanzapine, quetiapine, ziprasidone, and aripiprazole, as first-line treatment for schizophrenia. These drugs can be uniformly characterized by their dual mode of action: in addition to antagonism of the dopamine D2receptor, they are also potent inhibitors at the serotonin 5-HT2Areceptor.

Cariprazine hydrochloride is a novel atypical antipsychotic that has antagonistic effect on dopamine D3 receptor, dopamine D2 receptor and 5-hydroxytryptamine 2B receptor. It can be used for treating schizophrenia and bipolar I disorder.

The chemical name of cariprazine hydrochloride is 3-[4-[2-[4-(2,3-dichlorophenyl)piperazin-1-yl]ethyl]cyclohexyl]-1,1-dimethylurea;hydrochloride and its chemical structure is shown in the Formula I:

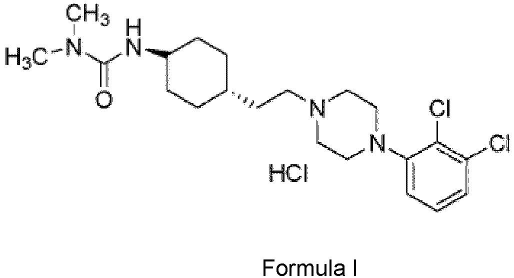

Cariprazine hydrochloride capsules are currently available on the market. This product is an oral preparation that should be taken daily to maintain its plasma concentration.

The patent EP4205745A1 discloses an aqueous suspension containing cariprazine, a preparation method and an application thereof. The pharmaceutical composition comprises: cariprazine pamoate solid particles, where a particle size of the cariprazine solid particles has a D(10) less than or equal to 30 microns, a D(50) less than or equal to 50 microns, and a D(90) less than or equal to 100 microns.

The stability of cariprazine is easily affected by external factors. It is known that stability in aqueous pharmaceutical form is achieved with extra excipients and difficult processes. We have developed a solid oral composition that is easier to use and does not require extra excipients and process steps as in aqueous formulations.

The patent CN117243955A discloses relates to the technical field of pharmaceutical preparations and discloses a cariprazine hydrochloride pharmaceutical composition and a capsule preparation containing the composition.

In the prior art documents, there was no invention that solved both the stability and dissolution profile problems at the same time. There is still a need for formulations that will give better results.

We have created a solid oral composition that provides the desired dissolution profile and stability using cariprazine hydrochloride with the particle size d (0.9) specified below.

### Detailed Description of the Invention

The main object of the present invention is to a solid oral composition comprising cariprazine hydrochloride has a d (0.9) particle size between 30 µm and 1 µm and at least one pharmaceutically acceptable excipient in a formulation providing the desired dissolution profile and stability.

Another object of the present invention is to provide a solid oral composition comprising cariprazine hydrochloride which is characterized by excellent pharmacotechnical properties, such as flowability and content uniformity.

Another object of the present invention is to provide a method for a solid oral composition comprising cariprazine hydrochloride prepared by simple, easy, time-saving and fast manufacturing methods.

Cariprazine undergoes hydrolytic degradation in the presence of excipients having high water activity and degradation of the active ingredient mainly depends upon the amount of free water released from the excipient, which is responsible for hydrolytic degradation. So, Cariprazine stability is affected by external factors easily. We saw that when we used cariprazine hydrochloride, a formulation with better stability was formed. Therefore, we used cariprazine hydrochloride salt in the formulation.

However, we encountered some problems in the dissolution profile when cariprazine hydrochloride was used. We have seen that these problems were overcome by using the active agent in d (0.9) particle sizes in the following proportions. Thus, we created a solid oral composition containing cariprazine hydrochloride, which has both stability and the desired dissolution profile.

As used herein, 'particle size' means the cumulative volume size distribution as tested by any conventionally accepted method such as the laser diffraction method (i.e. malvern analysis). The term d (0.9) means, the size at which 90% by volume of the particles are finer. The term d (0.5) means, the size at which 50% by volume of the particles are finer.

According to one embodiment, a solid oral composition comprising cariprazine hydrochloride and at least one pharmaceutically acceptable excipient, wherein cariprazine hydrochloride has a d (0.9) particle size between 30 µm and 1 µm.

According to one embodiment, cariprazine hydrochloride has a d (0.9) particle size between 30 µm and 2 µm, between 25 µm and 3 µm, between 20 µm and 3 µm, between 15 µm and 4 µm, between 10 µm and 1 µm.

According to one embodiment of the present invention, the amount of cariprazine hydrochloride is between 0.1% and %10.0 by weight in the total composition.

According to one embodiment of the present invention, the composition further comprises at least one filler which are selected from the group comprising microcrystalline cellulose, lactose, lactose monohydrate, spray-dried lactose monohydrate, anhydrous lactose, mannitol, maltodextrin, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, sugar sphericals, sulfobutylether beta-cyclodextrin, tragacanth, trehalose, polysorbate 80 or mixtures thereof.

According to one embodiment of the present invention, the filler is microcrystalline cellulose.

According to this embodiment of the present invention, the amount of filler is between 80.0% and 98.0% by weight of the total composition. Preferably, the amount of filler is between 90.0% and 96.0% by weight of the total composition. The amount provides the desired flowability and content uniformity.

According to one embodiment of the invention, a solid oral composition comprises at least one pharmaceutically acceptable excipient is selected from the group comprising binders, lubricants or mixtures thereof.

Suitable binders are selected from the group comprising hydroxypropyl cellulose, corn starch, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binder is between 0.5% and 10.0% by weight of the total composition. Preferably, the amount of binder is between 1.0% and 5.0% by weight of the total composition.

According to this embodiment of the present invention, the binder is hydroxypropyl cellulose.

Suitable lubricants are selected from the group comprising anhydrous colloidal silicon dioxide, magnesium stearate, sodium stearyl fumarate, magnesium oxide, silicone dioxide, talc, polyethylene glycol, stearic acid, aluminum silicate, magnesium silicate, colloidal silica, or mixtures thereof.

According to an embodiment of the present invention, the lubricant is magnesium stearate.

According to this embodiment of the present invention, the amount of lubricant is between 0.01% and 5.0% by weight of the total composition.

According to an embodiment of the present invention, the solid oral composition is in the form of tablet or capsule.

According to an embodiment of the present invention, the solid oral composition comprises;
a) Cariprazine hydrochloride having a d (0.9) particle size between 30 µm and 1 µm
b) Microcrystalline Cellulose
c) Hydroxypropyl cellulose
d) Magnesium stearate

A process for preparing the solid oral composition comprising cariprazine hydrochloride comprises the following steps;
a. sifting cariprazine hydrochloride having a d (0.9) particle size between 30 µm and 1 µm and at least one binder,
b. sifting of at least one filler,
c. geometric mixing of the compounds in steps (a) and (b),
d. sifting the mixture in step (c),
e. sifting the at least one lubricant, adding it to the mixture in step (d) and mixing,
f. filling capsules,
g. packaging with aluminum blister.

A process for preparing the solid oral composition comprising cariprazine hydrochloride comprises the following steps;
h. sifting cariprazine hydrochloride having a d (0.9) particle size between 30 µm and 1 µm and hydroxypropyl cellulose,
i. sifting of microcrystalline cellulose,
j. geometric mixing of the compounds in steps (a) and (b),
k. sifting the mixture in step (c),
l. sifting the magnesium stearate, adding it to the mixture in step (d) and mixing,
m. filling capsules,
n. packaging with aluminum blister.

### Example 1; Capsule composition

| **Ingredients** | **% by weight** | **% by weight** | **% by weight** | **% by weight** | **% by weight** |
|---|---|---|---|---|---|
| Cariprazine hydrochloride | 1.21 | 2.41 | 3.62 | 4.82 | 0.1-10 |
| Microcrystalline Cellulose | 95.28 | 94.07 | 92.86 | 91.66 | 80-98 |
| Hydroxypropyl cellulose | 2.78 | 2.78 | 2.78 | 2.78 | 0.5-10 |
| Magnesium stearate | 0.74 | 0.74 | 0.74 | 0.74 | 0.01-5 |
| **Total Weight** | 100 | 100 | 100 | 100 | 100 |

A process for example 1;
a) sifting cariprazine hydrochloride and hydroxypropyl cellulose together at 850 microns,
b) sifting of microcrystalline cellulose through 600 micron sieve,
c) geometric mixing of the compounds in steps (a) and (b) with 5-step mixing,
d) sifting the mixture in step (c) through 850 micron sieve,
e) sifting magnesium stearate through a 600 micron sieve, adding to the mixture in step (d) and mixing,
f) filling capsules,
g) packaging with aluminum blister.

## Claims

1. A solid oral composition comprising cariprazine hydrochloride and at least one pharmaceutically acceptable excipient, wherein cariprazine hydrochloride has a d (0.9) particle size between 30 µm and 1 µm.

2. The solid oral composition according to claim 1, wherein cariprazine hydrochloride has a d (0.9) particle size between 30 µm and 2 µm, between 25 µm and 3 µm, between 20 µm and 3 µm, between 15 µm and 4 µm, between 10 µm and 1 µm.

3. The solid oral composition according to claim 1, wherein the amount of cariprazine hydrochloride is between 0.1% and %10.0 by weight in the total composition.

4. The solid oral composition according to claim 1, wherein the composition further comprises at least one filler which are selected from the group comprising microcrystalline cellulose, lactose, lactose monohydrate, spray-dried lactose monohydrate, anhydrous lactose, mannitol, maltodextrin, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, sugar sphericals, sulfobutylether beta-cyclodextrin, tragacanth, trehalose, polysorbate 80 or mixtures thereof.

5. The solid oral composition according to claim 4, wherein the filler is microcrystalline cellulose.

6. The solid oral composition according to claim 4 or 5, wherein the amount of filler is between 80.0% and 98.0% by weight of the total composition.

7. The solid oral composition according to claim 1, wherein at least one pharmaceutically acceptable excipient is selected from the group comprising binders, lubricants or mixtures thereof.

8. The solid oral composition according to claim 7, wherein binders are selected from the group comprising hydroxypropyl cellulose, corn starch, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

9. The solid oral composition according to claim 7 or 8, wherein the binder is hydroxypropyl cellulose.

10. The solid oral composition according to claim 8 or 9, wherein the amount of binders is between 0.5% and 10.0% by weight of the total composition.

11. The solid oral composition according to claim 7, wherein lubricants are selected from the group comprising anhydrous colloidal silicon dioxide, magnesium stearate, sodium stearyl fumarate, magnesium oxide, silicone dioxide, talc, polyethylene glycol, stearic acid, aluminum silicate, magnesium silicate, colloidal silica, or mixtures thereof.

12. The solid oral composition according to claim 7 or 11, wherein the lubricant is magnesium stearate.

13. The solid oral composition according to claim 11 or 12, wherein the amount of lubricant is between 0.01% and 5.0% by weight of the total composition.

14. The solid oral composition according to any preceding claim, wherein the solid oral composition is in the form of tablet or capsule.

15. The solid oral composition according to any preceding claim, wherein the solid oral composition comprises;
a. Cariprazine hydrochloride
b. Microcrystalline Cellulose
c. Hydroxypropyl cellulose
d. Magnesium stearate
